# EUROPÄISCHE PATENTSCHRIFT

(11) **EP 1 683 531 B1**
(45) Veröffentlichungstag und Bekanntmachung des Hinweises auf die Patenterteilung: **12.12.2012**
(21) Anmeldenummer: 05028458.7
(22) Anmeldetag: 24.12.2005
(51) Int. Cl.: A61L 27/28, A61L 27/54

(54) **Antibiotische Beschichtung von Implantaten**
Antibiotic coating of implants
Revêtement antibiotique des implants

(30) Priorität: 19.01.2005 DE 102005002703
(43) Veröffentlichungstag der Anmeldung: 26.07.2006
(73) Patentinhaber: Heraeus Kulzer GmbH, 63450 Hanau (DE)
(72) Erfinder: Kühn, Klaus-Dieter, 35041 Marburg (DE); Vogt, Sebastian, 99084 Erfurt (DE); Schnabelrauch, Matthias, 07749 Jena (DE)
(74) Vertreter: Kühn, Hans-Christian

(56) Entgegenhaltungen:
- WO-A-03/039612
- DE-A1- 10 254 215
- GB-A- 1 090 421

## Beschreibung

Gegenstand der Erfindung ist eine antibiotische Beschichtung von Implantaten und Verfahren zu deren Herstellung.

Der Begriff Implantat charakterisiert Materialien und Vorrichtungen, die im Zuge eines chirurgischen Eingriffes mindestens teilweise im Körperinneren eingebracht werden. Diese Implantate können im Kontakt zum Knochen und anderen Elementen des Stütz- und Bewegungsapparates sowie auch in Kontakt mit Blut oder Bindegewebe stehen. In der Unfallchirurgie und in der Orthopädie werden in großem Umfang unterschiedlichste Osteosynthesematerialien, Endoprothesen und auch Knochenersatzwerkstoffe eingesetzt. Problematisch ist jedoch, dass sich an der Grenzfläche zwischen dem Implantat und dem umliegenden Gewebe mikrobielle Keime ansiedeln und schwerwiegende Infektionen verursachen können. Die implantatassoziierte Infektion im Knochengewebe gehört zu den schwersten Komplikationen bei der Verwendung von lmplantatmaterialien im Knochengewebe. Die implantatassoziierte Infektion ist sehr kompliziert und zeitaufwendig zu behandeln. Damit sind erhebliche Kosten verbunden. Es ist daher sinnvoll, Implantatmaterialien insbesondere in den für eine Infektion besonders kritischen ersten Tagen nach der Implantation gegenüber einer Keimbesiedlung durch die lokale Freisetzung von antimikrobiellen Agenzien an der Implantatoberfläche zu schützen.

In der Gefäßchirurgie finden Prothesen aus gestrecktem PTFE und aus gewebten Polyestern vielfältige Anwendung zum Ersatz von Blutgefäßen. Bei der Implantation dieser Gefäßprothesen besteht die Gefahr, dass in den ersten Stunden bis Tagen nach der Implantation Keime in das Prothesenmaterial einwandern und sich auf der inneren Oberfläche des Prothesenmaterials ansiedeln können. Deshalb erscheint es auch hier sinnvoll , einen temporären antibiotischen Schutz auf das Prothesenmaterial aufzubringen.

Zum Schutz von Implantoberflächen wurde eine Reihe von antibiotischen Beschichtungen auf der Grundlage von resorbierbaren Polymeren vorgeschlagen.

Im EP 0328421 wird eine Zusammensetzung offenbart, die aus einem Matrix-bildenden Polymer aus der Gruppe der Polyurethane, der Silikone und der biodegradierbaren Polymere besteht. In diesen Matrixbildnern ist eine synergistische Kombination eines Silbersalzes mit Chlorhexidin enthalten.

Im EP 0652017 wird eine Beschichtung von Biomaterialien dargelegt, welche die Blutgerinnung und das Anhaften von plasmatischen und zellulären Blutbestandteilen an dem beschichteten Biomaterial verhindern soll. Diese Beschichtung ist selbsthaftend auf der Biomaterialoberfläche und wird im Körper permanent degradiert. Die Beschichtung ist im wesentlichen aus Poly-α-hydroxycarbonsäuren, wie Polymilchsäuren, aufgebaut.

Ähnliche technische Lösungen auf der Grundlage von degradierbaren Polyestern wurden in den Schriften WO 00/15273, US 3277003, US 3839297, US 5378540, US 5312437, US 5123912, US 5100433, US 5032638, US 4857602 und US 4711241 offenbart. Problematisch ist bei den Beschichtungen, die biologisch degradierbare Polymere enthalten, dass der Polymerabbau relativ langsam erfolgt und dabei insbesondere bei Beschichtung von Implantatmaterialien, die im Knochengewebe einwachsen sollen, eine Barrierewirkung gegenüber dem einwachsenden Knochengewebe auftreten kann. Kritisch muss auch die Bildung von aciden Degradationsprodukten, wie Milchsäure und Glykolsäure, gesehen werden, die bei einer lokalen Ansammlung entzündliche Prozesse verursachen können. Ein weiteres Problem kann bei polymeren Beschichtungen darin bestehen, dass bei einer Scherbeanspruchung, wie sie bei Press-Fit-Techniken typischerweise auftritt, die gesamte Beschichtung als Film abgezogen oder abgerollt werden kann.

Im EP 0279666 wird eine Beschichtung von chirurgischen Fäden beschrieben. Bei dieser Beschichtung handelt es sich um Sucrosefettsäurester.

In US 4532929 wird eine trockene Beschichtung von chirurgischen Fäden beschrieben, die auf der Verwendung von Erdalkalifettsäuresalzen beruht. Diese sollen hauptsächlich eine Wirkung als Schlichtmittel haben.

Im WO 0007574 wird ein nichtabbaubares Medizinprodukt vorgeschlagen, das eine Substanz A und eine Substanz B umfasst, wobei die Substanz A lipophiler ist als die Substanz B ist und die Substanz A in Wasser löslicher ist als die Substanz B. Die Substanzen A und B sind dabei bevorzugt pharmazeutische Wirkstoffe. Bevorzugt ist auch, dass nicht-ionische Surfactants als Substanz A bevorzugt sind.

Die Aufgabe der Erfindung besteht darin, eine temporäre antibiotische Implantatbeschichtung zu entwickeln, die in kostengünstiger, wirtschaftlich vorteilhafter Weise auf unterschiedlichste Implantatmaterialien aufgebracht werden kann. Diese Beschichtung soll so beschaffen sein, dass einerseits wirksame Antibiotika-Mengen lokal an der Grenzfläche zwischen dem Implantat und dem Gewebe freigesetzt werden können und dass andererseits die Beschichtung vom angrenzenden humanen Gewebe innerhalb kurzer Zeit ohne Freisetzung toxischer oder acider Zersetzungsprodukte abgebaut werden kann. Wichtig ist diese Eigenschaft insbesondere bei Endoprothesen, wie zum Beispiel die nicht zementierten Hüftgelenksendoprothesen. Das Einwachsen des Knochengewebes in die porösen oder aufgerauhten Oberflächenstrukturen der nicht-zementierten Endoprothesen darf weder durch eine länger andauernde Barrierewirkung noch durch toxische Abbauprodukte der antibiotischen Beschichtung behindert werden, um eine optimale Funktion der Endoprothesen nicht zu beeinträchtigen. Eine weitere Aufgabe besteht darin, dass die zu entwickelnde Beschichtung auf der Implantatoberfläche haftet und dass auch nach einer Scherbeanspruchung, wie sie beim Einsetzen von Implantaten häufig auftritt, die Beschichtung nicht als Film abgezogen oder abgerollt wird. Die Beschichtung soll auch nach einer Scherbanspruchung noch zum größten Teil erhalten bleiben und einen antibiotischen Schutz gewährleisten können.

Die Aufgabe wurde gelöst durch die antibiotische Beschichtung gemäß Anspruch 1. Diese antibiotische Beschichtung von Implantaten besteht aus einem Matrixbildner, in dem ein Additiv gelöst ist, und einem Antibiotikum oder mehreren Antibiotika, wobei in dem Gemisch aus Matrixbildner und Additiv ein Antibiotikum/Antibiotika suspendiert ist und/oder ein mit dem Gemisch aus Matrixbildner und Additiv mischbares Antibiotikum/Antibiotika gelöst ist, wobei der Matrixbildner aus der Gruppe ausgewählt ist, die aus Glycerintristearat, Glycerintripalmitat, Glycerintrimyristat, Glycerintribehenat, Stearinsäure, Palmitinsäure, Myristinsäure, Behensäure, Myristylpalmitat, Cetylpalmitat und Cerylcerotinat besteht, und das Additiv aus der Gruppe ausgewählt ist, die aus Stearinsäure, Palmitinsäure und Myristinsäure besteht.

Unter dem Begriff "gesättigt" werden Verbindungen verstanden, die keine Doppel- oder Dreifachbindungsysteme enthalten. Unter dem Begriff "niedermolekular" wird die Eigenschaft des Matrixbildners verstanden, dass seine Molmasse kleiner 1000 g/mol ist. Der Begriff degradierbar ist hier so definiert, dass der degradierbare Matrixbildner durch die im humanen oder tierischen Organismus üblicherweise vorhandenen Enzyme und Enzymsysteme, wie Lipasen, die Enzymsysteme der β-Oxidation, der Glykolyse und des Citronensäurecyclus abgebaut werden können. Unter dem Begriff "hydrophobes, niedermolekulares Additiv" werden organische, hydrophobe Moleküle mit einer Molmasse kleiner 1000 g/mol verstanden, welche die Adhäsion der Beschichtung auf der Implantatoberfläche im Sinne einer besseren Haftung beeinflussen.

Vorzugsweise liegt die Beschichtung im Temperaturbereich von 20°C bis 45 °C im festen Aggregatzustand vor und kann durch Druckkräfte und Scherkräfte plastisch verformt werden. Die plastische Verformbarkeit ist eine sehr vorteilhafte Eigenschaft der Beschichtung. Dadurch ist es ausgeschlossen, dass scharfkantige Splitter oder Partikel bei der Implantation aus der Beschichtung ablöst werden können, die eventuell mechanische Reizungen hervorrufen können. Vor allem bei einer Scherbeanspruchung, wie sie bei der Implantation von zementfreien Hüftendoprothesen auftreten, wird die Beschichtung deformiert und drückt sich in die rauhen Oberflächenstrukturen der Prothese und in das umliegende spongiöse Knochengewebe ein.

Der Matrixbildner ist aus Glycerintristearat, Glycerintripalmitat, Glycerintrimyristat, Glycerintribehenat, Stearinsäure, Palmitinsäure, Myristinsäure, Behensäure, Myristylpalmitat, Cetylpalmitat, Cerylcerotinat und Glycerintriester, die unterschiedliche, geradzahlige Fettsäuren enthalten, aufgebaut. Die Glycerintriester stellen gesättigte Fette dar, die überraschend gut auf metallischen und nichtmetallischen Oberflächen haften. Im humanen Organismus stellen die Fette hauptsächlich Glycerinester der Palmitinsäure, der Stearinsäure und der Ölsäure dar. Daneben sind in geringeren Mengen noch andere Fettsäuren in den Fetten enthalten. Die erfindungsgemäßen Matrixbildner sind daher dem humanen Fett sehr ähnlich. Der Knochen, insbesondere die Spongiosa, enthält selbst Fette. Die erfindungsgemäßen Fette können durch die im humanen Organismus vorhandenen Stoffwechselwege zum Fettabbau problemlos abgebaut werden. Dadurch ist die Bildung von toxischen oder aciden Abbauprodukten, wie sie bei der Verwendung von Polymilchsäuren und Polyglykolsäuren auftreten, ausgeschlossen. Ein besonderer Vorteil besteht darin, dass der enzymatische Fettabbau deutlich schneller erfolgt als die hydrolytische Zersetzung von degradierbaren Polyestern. Durch die Verwendung von gesättigten Glycerintriestern ist auch die Gefahr der Bildung von Zersetzungsprodukten, wie sie bei ungesättigten Fetten entstehen, weitgehend vermieden. Der Matrixbildner sollte immer in nur geringen Mengen, in sehr dünnen Schichten, auf die Implantatoberflächen aufgebracht werden, um das Risiko der Entstehung von Fettembolien zu vermeiden.

Erfindungsgemäß ist weiterhin, dass Stearinsäure, Palmitinsäure und Myristinsäure als niedermolekulares, hydrophobes Additiv eingesetzt werden. Diese Substanzen haften sehr gut auf Metalloberflächen und auf Kunststoffoberflächen.

Es ist zweckmäßig, Gentamicinsulfat, Tobramycinsulfat, Amikacinsulfat, Netilmicinsulfat, Sisomycinsulfat, Vancomycinhydrochlorid, Teicoplanin, Ramoplanin, Clindamycinhydrochlorid, Lincomycinhydrochlorid, Metronidazol, Tinidazol, Gentamicinpalmitat, Gentamicinmyristat, Gentamicinlaurat, Tobramycinpalmitat, Tobramycinmyristat, Amikacinpalmitat, Amikacinmyristat, Amikacinlaurat, Linezolid, Chlorhexidinstearat, Chlorhexidinpalmitat, Chlorhexidinlaurat, Griseofulvin, Nytatin, Fuconazol, Moxifloxazol, Ciprofloxacin, Fusidinsäure, Rifampicin, Rifamycin, Fosfomycin, Cycloserin, Polyhexanid und Trichlosan als Antibiotika bevorzugt werden. Die aufgeführten Laurate, Myristate und Palmitate der Antibiotika sind die Fettsäuresalze der entsprechenden Antibiotika und nicht die Fettsäureester der Antibiotika. Unter dem Begriff Antibiotika werden auch vereinfacht die Antiseptika wie Chlorhexidin, Polyhexanid und Trichlosan mit verstanden. Im Sinne der Erfindung ist ebenfalls, dass zusätzlich zu den Antibiotika auch Wachstumsfaktoren, wie BMP2 und BMP 7, und Hormone, wie Calcitonin, mit in der antibiotischen Beschichtung enthalten sein können. Ebenso ist es möglich, dass zusätzlich Bisphosphonate, wie Zoledronat oder Ibandronat, in der erfindungsgemäßen Beschichtung integriert sind. Weiterhin ist erfindungsgemäß, dass die antibiotische Beschichtung bevorzugt aus 1,0-98,0 Masseprozent aus einem gesättigtem, organischen, hydrophoben, niedermolekularen Matrixbildner, der ein Schmelzpunkt im Temperaturbereich von 45 °C bis 100 °C hat, 0,1-5,0 Masseprozent niedermolekulares, hydrophobes Additiv und 0,1-5,0 Masseprozent Antibiotikum/Antibiotika gebildet ist.

Die Erfindung betrifft auch ein Verfahren zur antibiotischen Beschichtung, das dadurch charakterisiert ist, dass ein Gemisch aus dem Matrixbildner, dem Additiv und dem Antibiotikum/Antibiotika auf eine Temperatur größer als der Schmelzpunkt des Matrixbildners erwärmt wird und dass in die gebildete Suspension oder in die homogene Schmelze das Implantat getaucht wird, wobei das Implantat zuvor auf eine Temperatur von mindestens 10 °C höher als der Schmelzpunkt des Matrixbildners temperiert wurde, und dass anschließend das beschichtete Implantat auf Raumtemperatur abgekühlt wird.

Erfindungsgemäß ist auch ein Verfahren zur antibiotischen Beschichtung, das dadurch charakterisiert ist, dass ein Gemisch aus dem Matrixbildner, dem Additiv und dem Antibiotikum/Antibiotika in einem organischen Lösungsmittel gelöst wird und dass anschließend die Lösung auf ein Implantat gesprüht wird, wobei das Substrat vor der Besprühung auf eine Temperatur von mindestens 10 °C höher als der Schmelzpunkt des Matrixbildners und mindestens 10 °C höher als der Siedepunkt des organischen Lösungsmittels temperiert wurde, und dass anschließend das beschichtete Implantat auf Raumtemperatur abgekühlt wird. Dem Verfahren liegt die Beobachtung zu Grunde, dass Gemische aus Fettsäuren und Antibiotika-Fettsäuresalzen in organischen Lösungen gelöst werden können und diese Lösungen auf Oberflächen aufgesprüht werden können. Es bilden sich überraschend dabei nur fest haftende Beschichtungen, wenn das Implantat zuvor auf eine Temperatur von mindestens 10 °C höher als der Siedepunkt des organischen Lösungsmittels wurde. Es zeigte sich weiterhin überraschend, das Gemische aus Fettsäuren und Antibiotika-Fettsäuresalzen anschmelzen können und dabei einen sehr fest haftenden Verbund mit unterschiedlichsten Materialien ausbilden können. Deshalb ist es sinnvoll, dass das zu beschichtende Implantat vor der Beschichtung eine Temperatur größer 10 °C als der Schmelzpunkt des Matrixbildners hat.

Außerdem ist ein Verfahren erfindungsgemäß, bei dem auf der Oberfläche von Implantaten aufgebrachte Gemische eines Matrixbildners, eines Additivs und eines Antibiotikums/Antibiotika durch Tempern auf eine Temperatur von mindestens dem Schmelzpunkt des Matrixbildners eine Beschichtung durch partielles oder vollständiges Aufschmelzen des Gemisches gebildet wird. So lassen sich zum Beispiel vorteilhaft auf gestreckten PTFE-Prothesen fest haftende Beschichtungen aus Antibiotika-Fettsäuresalzen/Fettsäuren aufbringen.

Weiterhin ist ein Verfahren zur antibiotischen Beschichtung ein Bestandteil der Erfindung, das dadurch charakterisiert ist, dass ein Gemisch aus dem Matrixbildner, dem Additiv und dem Antibiotikum/Antibiotika zu einem festen kompakten Körper geformt wird und dass der Körper auf die Oberfläche des Implantats gerieben wird und sich dadurch eine Beschichtung auf der Implantatoberfläche abscheidet und dass gegebenenfalls das beschichtete Implantat auf eine Temperatur von mindestens dem Schmelzpunkt des Matrixbildners erwärmt wird. Der Körper aus dem Gemisch aus dem Matrixbildner, dem Additiv und dem Antibiotikum/Antibiotika wird beispielsweise ähnlich wie ein konventioneller Klebestift verwendet. Der Körper wird auf die zu beschichtende Oberfläche gestrichen. Dabei bildet sich eine Beschichtung. Diese Beschichtung kann durch Erwärmung über den Schmelzpunkt des Matrixbildners partiell aufgeschmolzen werden. Dadurch erhält die Beschichtung eine glattere Oberfläche und der Verbund der Beschichtung mit der Implantatoberfläche wird verbessert.

Die Erfindung wird durch nachstehende Beispiele erläutert, ohne jedoch die Erfindung zu beschränken.

### Beispiel 1:

Es werden 74,60 g Tripalmitin (ein Gemisch aus Glycerintripalmitat und Glycerintristearat) (Fluka), 0,10 g Palmitinsäure (Fluka) und 25,30 g Gentamicinsulfat (AK 640) intensiv miteinander vermahlen. Dieses Gemisch wird bei 80°C unter Rühren aufgeschmolzen. Es entsteht eine milchige, dünnflüssige Suspension. In diese Suspension wird eine auf 100 °C temperierte, sandgestrahlte Titanscheibe (TiAl6V4, d= 20 mm) getaucht. Nach 3 Sekunden wird die Titanscheibe entnommen. Nach Abkühlung auf Raumtemperatur hat sich eine transparente, wachsartige Beschichtung gebildet. Die Masse der Beschichtung beträgt 32,5 mg (5,1 mg Gentamicinbase).

### Beispiel 2:

Es werden 5,00 g Gentamicinpentakispalmitat (Palmitinsäuresalz des Gentamicins), 0,80 g Palmitinsäure, 0,10 g Stearinsäure in 100,00 g Methanol gelöst. Es entsteht eine sichtklare Lösung. Ein Edelstahlzylinder (d= 10 mm, h= 100 mm) wird auf 90 °C erwärmt. Auf diesen Edelstahlzylinder wird die zuvor hergestellte methanolische Lösung von Gentamicinpalmitat/Palmitinsäure/Stearinsäure aufgesprüht. Es bildet sich unter Verdampfung des Lösungsmittel und unter Verfilmung des abgeschiedenen Gemisches eine glasartige, transparente, fest haftende Beschichtung (m= 88 mg) aus.

### Beispiel 3:

Es werden 74,60 g Tripalmitin (ein Gemisch aus Glycerintripalmitat und Glycerintristearat) (Fluka), 0,10 g Palmitinsäure (Fluka) und 25,30 g Gentamicinsulfat (AK 640) intensiv miteinander vermahlen. Dieses Gemisch wird bei 80°C unter Rühren aufgeschmolzen. Es entsteht eine milchige, dünnflüssige Suspension. Nach abkühlen auf Raumtemperatur entsteht ein wachsartiger, weißer Festkörper. Dieser wird aufgemahlen. Das entstandene feine Pulver wird auf einen Edelstahlzylinder (d= 10 mm , h= 100 mm) durch Sprühen mit Druckluft aufgebracht. Die Pulverpartikel haften sehr lose an der Metalloberfläche. Anschließend wird der Metallzylinder auf 80 °C erwärmt. Dabei schmilzt das aufgebrachte Pulver an und ein gleichmäßige Beschichtung (m= 96 mg) bildet sich aus.

### Beispiel 4:

Ein Edelstahlzylinder (d= 10 mm, h= 100 mm) wird auf 80 °C erwärmt und danach für 3 Sekunden in ein Pulverbett getaucht, das aus einem pulverförmigen, homogenen Gemisch (Korngröße < 250 µm) aus 25,00 g Gentamicinpentakispalmitat (Palmitinsäuresalz des Gentamicins), 4,00 g Palmitinsäure, 0,50 g Stearinsäure besteht. Nach Entnahme des Edelstahlzylinders aus dem Pulverbett hat sich eine Schicht von angeschmolzenen Pulver and die Zylinderoberfläche angelagert. Der Edelstahlzylinder wird danach für 15 Minuten bei 80 °C getempert, wobei sich eine gut haftende Beschichtung (m=125 mg) ausbildet.

### Beispiel 5:

Es werden 5,00 g Gentamicinpentakispalmitat (Palmitinsäuresalz des Gentamicins), 0,80 g Palmitinsäure, 0,10 g Stearinsäure in 100,00 g Methanol gelöst. Es entsteht eine sichtklare Lösung. In diese Lösung wird eine PTFE-Prothese (Länge 10 cm) getaucht. Nach Verdampfen des Lösungsmittels hat sich eine dünne Schicht (m= 39,5 mg) ausgebildet. Die beschichtete PTFE-Prothese wird 10 Minuten bei 80°C im Trockenschrank gelagert. Dabei schmilzt die Beschichtung partiell auf und eine gleichmäßige, festhaftende Beschichtung bildet sich aus.

### Beispiel 6:

Es werden 5,00 g Gentamicinpentakispalmitat (Palmitinsäuresalz des Gentamicins), 0,80 g Palmitinsäure, 0,10 g Stearinsäure in 100,00 g Methanol gelöst. Es entsteht eine sichtklare Lösung. In diese Lösung wird ein quadratischer PGA-Filz (30 mm x 30 mm) getaucht. Nach Verdampfen des Lösungsmittels hat sich eine dünne Schicht (m= 35,2 mg) ausgebildet. Der beschichtete PGA-Filz wird 10 Minuten bei 80°C im Trockenschrank gelagert. Dabei schmilzt die Beschichtung partiell auf und eine gleichmäßige, festhaftende Beschichtung bildet sich aus.

### Beispiel 7:

Es werden 74,60 g Tripalmitin (ein Gemisch aus Glycerintripalmitat und Gglycerintristearat) (Fluka), 0,10 g Palmitinsäure (Fluka) und 25,30 g Gentamicinsulfat (AK 640) intensiv miteinander vermahlen. Dieses Gemisch wird bei 80°C unter Rühren aufgeschmolzen. Es entsteht eine milchige, dünnflüssige Suspension. Diese Suspension wird in eine zylinderförmige Form (d= 10 mm, h= 10 mm) gegossen. Nach Abkühlung auf Raumtemperatur ist ein weißer, wachsartiger Zylinder entstanden. Dieser Zylinder wird auf eine sandgestrahlte Titanscheibe (TiA16V4, d= 15 mm) gerieben. Es bildet sich eine wachsartige, noch relativ ungleichmäßige Beschichtung aus. Die beschichtete Titanscheibe wird dann für 15 Minuten bei 80 °C im Trockenschrank gelagert. Dabei erfolgt ein partielles Aufschmelzen und eine gleichmäßige Beschichtung entsteht. Die Beschichtung hat eine Masse von 15,8 mg (2,55 mg Gentamicinbase-Gehalt).

## Patentansprüche

1. Antibiotische Beschichtung von Implantaten, bestehend aus einem Matrixbildner, in dem ein Additiv gelöst ist, und einem Antibiotikum oder mehreren Antibiotika, wobei in dem Gemisch aus Matrixbildner und Additiv ein Antibiotikum/Antibiotika suspendiert ist und/oder ein mit dem Gemisch aus Matrixbildner und Additiv mischbares Antibiotikum/Antibiotika gelöst ist,
**dadurch gekennzeichnet, dass** der Matrixbildner aus der Gruppe ausgewählt ist, die aus Glycerintristearat, Glycerintripalmitat, Glycerintrimyristat, Glycerintribehenat, Stearinsäure, Palmitinsäure, Myristinsäure, Behensäure, Myristylpalmitat, Cetylpalmitat und Cerylcerotinat besteht, und das Additiv aus der Gruppe ausgewählt ist, die aus Stearinsäure, Palmitinsäure und Myristinsäure besteht.

2. Antibiotische Beschichtung von Implantaten nach Anspruch 1, **dadurch gekennzeichnet, dass** die Beschichtung im Temperaturbereich von 20°C bis 45 °C im festen Aggregatzustand vorliegt und durch Druckkräfte und Scherkräfte plastisch verformt werden kann.

3. Antibiotische Beschichtung von Implantaten nach Anspruch 1 oder 2, **dadurch gekennzeichnet, dass** Gentamicinsulfat, Tobramycinsulfat, Amikacinsulfat, Netilmicinsulfat, Sisomycinsulfat, Vancomycinhydrochlorid, Teicoplanin, Ramoplanin, Clindamycinhydrochlorid, Lincomycinhydrochlorid, Metronidazol, Tinidazol, Gentamicinpalmitat, Gentamicinmyristat, Gentamicinlaurat, Tobramycinpalmitat, Tobramycinmyristat, Amikacinpalmitat, Amikacinmyristat, Amikacinlaurat, Linezolid, Chlorhexidinstearat, Chlorhexidinpalmitat, Chlorhexidinlaurat, Griseofulvin, Nytatin, Fuconazol, Moxifloxazol, Ciprofloxacin, Fusidinsäure, Rifampicin, Rifamycin, Fosfomycin, Cycloserin, Polyhexanid und Trichlosan als Antibiotika eingesetzt werden.

4. Antibiotische Beschichtung von Implantaten nach einem der Ansprüche 1 bis 3, **dadurch gekennzeichnet, dass** die Beschichtung bevorzugt aus 1,0 - 98,0 Masseprozent aus mindestens einem Matrixbildner, 0,1-5,0 Masseprozent Additiv und 0,1-5,0 Masseprozent Antibiotikum/Antibiotika gebildet ist.

5. Verfahren zur antibiotischen Beschichtung von Implantaten, **dadurch gekennzeichnet, dass** ein Gemisch aus Matrixbildner, Additiv und Antibiotikum/Antibiotika nach einem der Ansprüche 1 bis 4 auf eine Temperatur größer als der Schmelzpunkt des Matrixbildners erwärmt wird und dass in die gebildete Suspension oder in die homogene Schmelze das Implantat getaucht wird, wobei das Implantat zuvor auf eine Temperatur von mindestens 10 °C höher als der Schmelzpunkt des Matrixbildners temperiert wurde, und dass anschließend das beschichtete Implantat auf Raumtemperatur abgekühlt wird.

6. Verfahren zur antibiotischen Beschichtung von Implantaten, **dadurch gekennzeichnet, dass** ein Gemisch aus Matrixbildner, Additiv und Antibiotikum/Antibiotika nach einem der Ansprüche 1 bis 4 in einem organischen Lösungsmittel gelöst wird und dass anschlie-βend die Lösung auf ein Implantat gesprüht wird, wobei das Implantat, vor der Besprühung auf eine Temperatur von mindestens 10 °C höher als der Schmelzpunkt des Matrixbildners und mindestens 10 °C höher als der Siedepunkt des organischen Lösungsmittels temperiert wurde, und dass anschließend das beschichtete Implantat auf Raumtemperatur abgekühlt wird.

7. Verfahren zur antibiotischen Beschichtung von Implantaten, bei dem ein Gemisch aus dem Matrixbildner, dem Additiv und dem Antibiotikum/Antibiotika nach einem der Ansprüche 1 bis 4 als Schicht auf die Implantatoberfläche aufgebracht wird, **dadurch gekennzeichnet, dass** das aufgebrachte Gemisch auf eine Temperatur von mindestens dem Schmelzpunkt des Matrixbildners erwärmt wird.

8. Verfahren zur antibiotischen Beschichtung von Implantaten, **dadurch gekennzeichnet, dass** ein Gemisch aus Matrixbildner, Additiv und Antibiotikum/Antibiotika nach einem der Ansprüche 1 bis 4 zu einem festen kompakten Körper geformt wird, dass der Körper auf die Oberfläche des Implantats gerieben wird und sich dadurch eine Beschichtung auf der Implantatoberfläche abscheidet und dass optional das beschichtete Implantat auf eine Temperatur von mindestens dem Schmelzpunkt des Matrixbildners erwärmt wird.

## Claims

1. Antibiotic coating of implants consisting of a matrix-forming substance, in which an additive is dissolved, and an antibiotic or multiple antibiotics, whereby an antibiotic/antibiotics is/are suspended in the mixture of matrix-forming substance and additive and/or an antibiotic/antibiotics that is/are miscible with the mixture of matrix-forming substance and additive is/are dissolved in the mixture of matrix-forming substance and additive,
**characterised in that** the matrix-forming substance is selected from the group consisting of glycerol tristearate, glycerol tripalmitate, glycerol trimyristate, glycerol tribehenate, stearic acid, palmitic acid, myristic acid, behenic acid, myristyl palmitate, cetyl palmitate, and cetyl cerotinate, and the additive is selected from the group consisting of stearic acid, palmitic acid, and myristic acid.

2. Antibiotic coating of implants according to claim 1, **characterised in that** the coating is in the solid state of aggregation in the temperature range from 20°C to 45°C and can be plastically deformed through pressure forces and shearing forces.

3. Antibiotic coating of implants according to claim 1 or 2, **characterised in that** gentamicin sulfate, tobramycin sulfate, amikacin sulfate, netilmicin sulfate, sisomycin sulfate, vancomycin hydrochloride, teicoplanin, ramoplanin, clindamycin hydrochloride, lincomycin hydrochloride, metronidazole, tinidazole, gentamicin palmitate, gentamicin myristate, gentamicin laurate, tobramycin palmitate, tobramycin myristate, amikacin palmitate, amikacin myristate, amikacin laurate, linezolid, chlorohexidine stearate, chlorohexidine palmitate, chlorohexidine laurate, griseofulvin, nystatin, fluconazole, moxifloxazole, ciprofloxacine, fusidinic acid, rifampicin, rifamycin, fosfomycin, cycloserine, polyhexanide, and triclosane are used as antibiotics.

4. Antibiotic coating of implants according to any one of the claims 1 to 3, **characterised in that** the coating is preferably made of 1.0 - 98.0 percent by mass of at least one matrix-forming substance, 0.1 - 5.0 percent by mass of additive, and 0.1 - 5.0 percent by mass of antibiotic/antibiotics.

5. Method for antibiotic coating of implants, **characterised in that** a mixture of matrix-forming substance, additive, and antibiotic/antibiotics according to any one of the claims 1 to 4 is heated to a temperature above the melting point of the matrix-forming substance and **in that** the implant is immersed into the suspension thus generated or into the homogeneously melted material, whereby the implant previously was equilibrated to a temperature at least 10 °C above the melting point of the matrix-forming substance, and **in that** the coated implant is subsequently cooled down to room temperature.

6. Method for antibiotic coating of implants, **characterised in that** a mixture of matrix-forming substance, additive, and antibiotic/antibiotics according to any one of the claims 1 to 4 is dissolved in an organic solvent and the solution is subsequently sprayed onto an implant, whereby the implant, prior to spraying, was equilibrated to a temperature at least 10 °C above the melting point of the matrix-forming substance and at least 10 °C above the boiling point of the organic solvent, and **in that** the coated implant is subsequently cooled down to room temperature.

7. Method for antibiotic coating of implants, in which a mixture of the matrix-forming substance, the additive, and the antibiotic/antibiotics according to any one of the claims 1 to 4 is applied to the implant surface as a layer, **characterised in that** the mixture that is applied is heated to a temperature that is at least equal to the melting point of the matrix-forming substance.

8. Method for antibiotic coating of implants, **characterised in that** a mixture of matrix-forming substance, additive, and antibiotic/antibiotics according to any one of the claims 1 to 4 is shaped into a compact body, **in that** the body is rubbed onto the surface of the implant and **in that** a coating is thus deposited on the implant surface, and **in that**, optionally, the coated implant is heated to a temperature that is at least equal to the melting point of the matrix-forming substance.

## Revendications

1. Revêtement antibiotique d'implants, constitué d'un formateur de matrice dans lequel un additif est dissous, et d'un antibiotique ou de plusieurs antibiotiques, dans lequel un antibiotique/des antibiotiques est/sont mis en suspension dans le mélange de formateur de matrice et d'additif et/ou un antibiotique/des antibiotiques pouvant être mélangé(s) au mélange de formateur de matrice et d'additif est/sont dissous,
**caractérisé en ce que** le formateur de matrice est sélectionné parmi le groupe qui se compose du tristéarate de glycérine, du tripalmitate de glycérine, du trimyristate de glycérine, du tribéhénate de glycérine, de l'acide stéarique, de l'acide palmitique, de l'acide myristique, de l'acide béhénique, du palmitate de myristyle, du palmitate de cétyle et du cérotinate de céryle, et additif est sélectionné parmi le groupe qui se compose de l'acide stéarique, de l'acide palmitique et de l'acide myristique.

2. Revêtement antibiotique d'implants selon la revendication 1, **caractérisé en ce que** le revêtement est présent à l'état d'agrégat solide dans la plage de température de 20°C à 45°C et peut être déformé de manière plastique par des forces de pression et des forces de cisaillement.

3. Revêtement antibiotique d'implants selon la revendication 1 ou 2, **caractérisé en ce que** du sulfate de gentamycine, du sulfate de tobramycine, du sulfate d'amikacine, du sulfate de nétilmicine, du sulfate de sisomycine, du chlorhydrate de vancomycine, de la teicoplanine, de la ramoplanine, du chlorhydrate de clindamycine, du chlorhydrate de lincomycine, du métronidazol, du tinidazol, du palmitate de gentamycine, du myristate de gentamycine, du laurate de gentamycine, du palmitate de tobramycine, du myristate de tobramycine, du palmitate d'amikacine, du myristate d'amikacine, du laurate d'amikacine, du linézolide, du stéarate de chlorhexidine, du palmitate de chlorhexidine, du laurate de chlorhexidine, de la griséofulvine, de la nystatine, du fluconazole, du moxifloxazole, de la ciprofloxacine, de l'acide fusidique, de la rifampicine, de la rifamycine, de la fosfomycine, de la cyclosérine, du polyhexanide et du triclosan sont utilisés en tant qu'antibiotiques.

4. Revêtement antibiotique d'implants selon l'une quelconque des revendications 1 à 3, **caractérisé en ce que** le revêtement est formé de préférence de 1,0 à 98,0 pourcent en masse d'au moins un formateur de matrice, de 0,1 à 5,0 pourcent en masse d'additif et de 0,1 à 5,0 pourcent en masse d'antibiotique(s).

5. Procédé pour l'enduction antibiotique d'implants, **caractérisé en ce qu'**un mélange de formateur de matrice, d'additif et d'antibiotique(s) selon l'une quelconque des revendications 1 à 4 est chauffé à une température supérieure au point de fusion du formateur de matrice et que l'implant est immergé dans la suspension formée ou dans la masse fondue homogène, dans lequel l'implant a été tempéré au préalable à une température d'au moins 10°C supérieure au point de fusion du formateur de matrice, et que l'implant enduit est ensuite refroidi à température ambiante.

6. Procédé pour l'enduction antibiotique d'implants, **caractérisé en ce qu'**un mélange de formateur de matrice, d'additif et d'antibiotique(s) selon l'une quelconque des revendications 1 à 4 est dissous dans un solvant organique et que la solution est ensuite pulvérisée sur un implant, dans lequel l'implant a été tempéré, avant la pulvérisation, à une température d'au moins 10°C supérieure au point de fusion du formateur de matrice et d'au moins 10°C supérieure au point d'ébullition du solvant organique, et que l'implant enduit est ensuite refroidi à température ambiante.

7. Procédé pour l'enduction antibiotique d'implants, lors duquel un mélange du formateur de matrice, de l'additif et du/des antibiotique(s) selon l'une quelconque des revendications 1 à 4 est appliqué comme couche sur la surface de l'implant, **caractérisé en ce que** le mélange appliqué est chauffé à une température d'au moins le point de fusion du formateur de matrice.

8. Procédé pour l'enduction antibiotique d'implants, **caractérisé en ce qu'**un mélange de formateur de matrice, d'additif et d'antibiotique(s) selon l'une quelconque des revendications 1 à 4 est moulé en un corps compact solide, que le corps est frotté à la surface de l'implant et qu'un revêtement se sépare à la surface de l'implant, et qu'en option, l'implant enduit est chauffé à une température d'au moins le point de fusion du formateur de matrice.
